(19) Europäisches Patentamt
European Patent Office
Office européen des brevets

(11) Publication number: 0 186 181
A2

(12) EUROPEAN PATENT APPLICATION

(21) Application number: 85116481.4

(22) Date of filing: 23.12.85

(51) Int. Cl.4: A61K 31/557 , //C07C177/00

(30) Priority: 24.12.84 US 685643
04.11.85 US 794912

(43) Date of publication of application:
02.07.86 Bulletin 86/27

(84) Designated Contracting States:
AT BE CH DE FR GB IT LI LU NL SE

(71) Applicant: SYNTEX (U.S.A.) INC.
3401 Hillview Avenue
Palo Alto, California 94304(US)

(72) Inventor: Sevelius, Hilli
58 Austin Avenue
Atherton California 94025(US)
Inventor: Schwartz, Kenneth E.
511 Virginia Avenue
San Mateo California 94402(US)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz
Siegfriedstrasse 8
D-8000 München 40(DE)

# (54) Uses for enprostil.

(57) A treatment for high levels of serum cholesterol, serum glucose, and GIP by giving orally to patients in need of such treatment a compound of the formula:

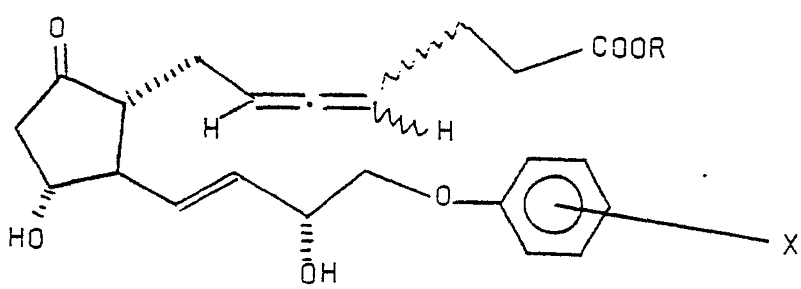

in form of a stereoisomer or mixture thereof, wherein R is hydrogen, or lower alkyl; X is hydrogen, halo, trifluoromethyl, lower alkyl or lower alkoxy, and the wavy lines represent either the α or β configuration, but one wavy line must be α when the other is β, or a pharmaceutically acceptable salt of the compound wherein R is hydrogen.

EP 0 186 181 A2

# USES FOR ENPROSTIL

This invention relates to new pharmaceutical uses for a class of prostaglandin compounds having the structure:

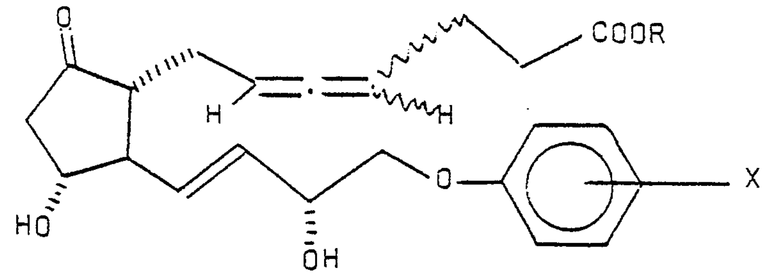

wherein R is hydrogen, or lower alkyl; X is hydrogen, halo, trifluoromethyl lower alkyl or lower alkoxy, and one wavy line is α when the other is β, and pharmaceutically acceptable salts thereof.

These compounds have now been found to be effective for lowering total serum cholesterol, LDL cholesterol, and apoprotein-B, and, surprisingly, delaying the time to and suppressing the peak levels of blood glucose, G.I.P, and insulin, and for lowering insulin levels without raising blood glucose.

The compounds of the present invention can be used to treat a variety of conditions where controlling levels of serum cholesterol, high levels of glucose, or high levels of serum insulin, or atherosclerosis would be beneficial. Examples of such conditions include hypercholesterolemia and other cardiovascular conditions, insulin dependent and non-insulin dependent diabetes mellitus, reactive hypoglycemia, and pancreatic islet cell hyperplasia (nesidioblastosis). Also the compounds of this invention are useful for the prevention and treatment of atherosclerosis.

United States Patent 4,459,310 issued to Dajani shows the use of 16-lower alkyl-16-hydroxy prostaglandins, such as misoprostol, to lower cholesterol blood levels in patients with hypercholesterolemia. The dosage form is not specified in this patent, nor is mention made of LDL cholesterol or apoproteins.

Konturek et al Prostaglandins, 15, 591-602 (1978) reported that 15S, 15 methyl PGE2 methyl ester administered by tube to the duodenum lowered insulin levels. It was noted, however, that there was no change in the serum glucose levels observed, despite lower insulin levels. Intravenous administration of 15S, 15 methyl PGE2 methyl ester similarly lowered insulin levels without affecting glucose levels. There was no change in the time to peak serum glucose levels following administration of the above identified 15S, 15 methyl PGE2 methyl ester either intraduodenally or intravenously.

A relative state of insulin resistance, associated with normal to elevated plasma insulin levels, is present in adults with non-insulin requiring diabetes mellitus. The cause of this condition is unknown but it has been associated with excessive weight gain. A prostaglandin induced lowering of circulating insulin levels might lead to decrease in insulin resistance and an improvement in the degree of diabetic control. Furthermore, in many of these individuals, elevated serum insulin levels are associated with an elevation in serum gastric inhibitory polypeptide (GIP), a peptide hormone found in the gastrointestinal mucosa which is widely thought to be one factor controlling the release of pancreatic insulin (Dupre et al., J. Clin. Endocrino Metab., 37, 826 (1973)). A reduction in serum GIP levels might also lower the degree of insulin resistance present in some individuals.

Furthermore, there is a similar surge in serum GIP levels in alimentary hypoglycemia, a condition characterized by a late surge in serum insulin following a carbohydrate meal and a state of relative hypoglycemia, (Hadji-Georgpoulos, et al. J. Clin. Endocrino Metab., 56, 648 (1983)).

A clinical problem present in the treatment of diabetes mellitus which must be treated with insulin administered subcutaneously, Type I diabetes, is that of coordinating the peak in serum insulin levels which occurs following subcutaneous administration of insulin with the peak in serum glucose levels following a meal. There may be some delay in insulin absorption and activity with the result that excessive surges in serum glucose may occur shortly after a meal. The conventional clinical approach to this problem is to administer subcutaneously a mixture of short-acting and long-acting insulins. There is no conventional method for delaying the time to onset of peak serum glucose following a meal.

A delay in the time to peak blood glucose levels or a lowering of the peak level that is ultimately achieved may improve diabetic control in both insulin requiring and non-insulin requiring diabetics.

The role of GIP in the pathogenesis of non-insulin dependent diabetes mellitus, Type II diabetes, is unclear. An elevation of GIP levels in association with the non-insulin dependent diabetic state has been described (Crockett, et al, Diabetes, 25 , 931-35 (1976)). The significance of our observations are unclear. However, "current understanding suggests that GIP may be an important enteric signal for the release of insulin in man . . . GIP may play a role in the pathogenesis of diabetes mellitus" (Crockett et al Diabetes, 25, 931-35, (1976)).

Previous methods of lowering GIP levels have included induction of weight loss, restriction of calorie intake, and administration of drugs to the patient which inhibit intestinal glucosidase activity. One such drug is Acarbose. See The Proceedings of the FirstInternational Symposium on Acarbose, 1982 Exerpta Medica, Princeton, New Jersey. Acarbose reduces postprandial serum glucose and insulin levels as well as postprandial GIP levels. There is no delay in the time to achieve peak glucose or insulin levels following a meal however, nor is there a change in total serum cholesterol. An improvement in the condition of patients with diabetes mellitus has been observed, however. This finding may be related to an inhibition of metabolism of complex sugars in the brush border in the intestinal lumen.

Both Type I and Type II diabetics are predisposed to premature atherosclerosis and cardiovascular disease. Certain other individuals show abnormal glucose tolerance following a meal, but are not overtly diabetic. These individuals may also have greater than average risk of these diseases, particularly if hypercholesterolemia is present.

This invention provides a method of reducing total serum cholesterol, LDL cholesterol and apoprotein-B delaying and suppressing peak serum glucose, insulin, and GIP levels in a disease state comprising:

administering an amount of a compound selected from the group consisting of

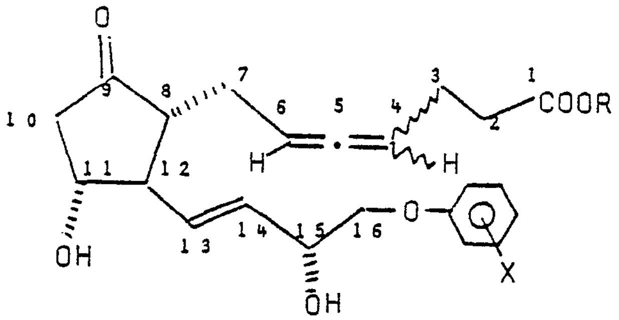

(I)

in form of a stereoisomer or mixture thereof, wherein R is hydrogen, or lower alkyl; X is hydrogen, halo, trifluoromethyl, lower alkyl or lower alkoxy, and the wavy lines represent either the α or β configuration, but one wavy line must be α when the other is β, or a pharmaceutically acceptable salt thereof.

This invention provides a method for lowering serum cholesterol levels, GIP, and insulin levels without raising glucose levels by administering a prostaglandin of the general formula:

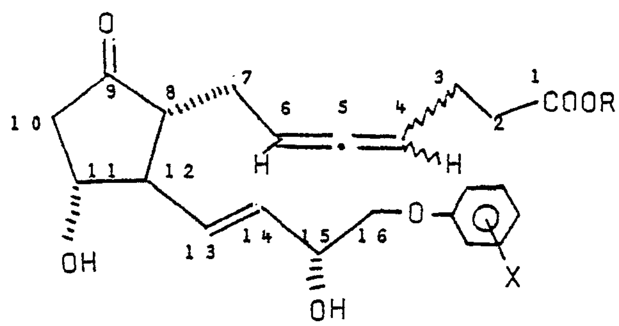

(I)

in form of a stereoisomer or mixture thereof, wherein R is hydrogen, or lower alkyl; X is hydrogen, halo, trifluoromethyl, lower alkyl or lower alkoxy, and the wavy lines represent either the α or β configuration, but one wavy line must be α when the other β, or a pharmaceutically acceptable salt thereof.

In particular, one especially preferred prostaglandin for use in the method of this invention is enprostil. Enprostil is the USAN name having the following structure:

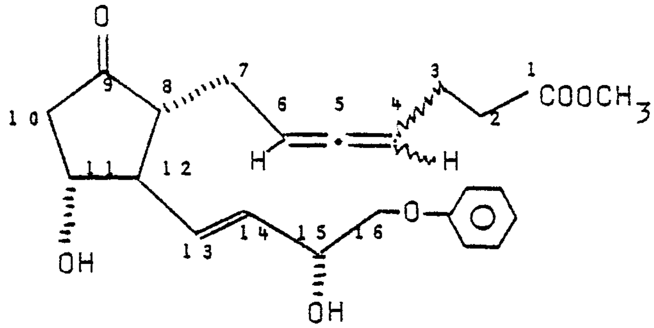

(I)

Enprostil is a mixture of four stereoisomers. They are:

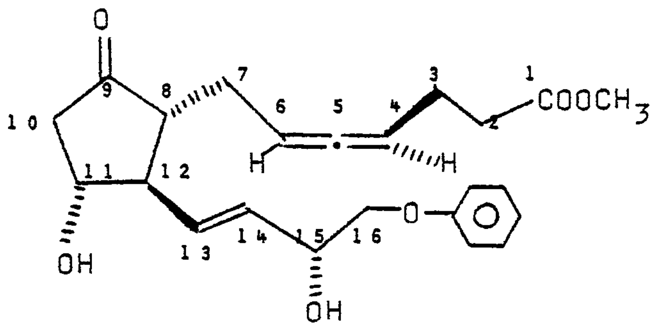

(Ia)

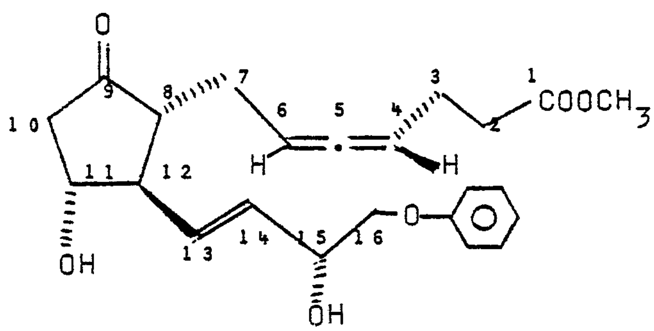

(Ia')

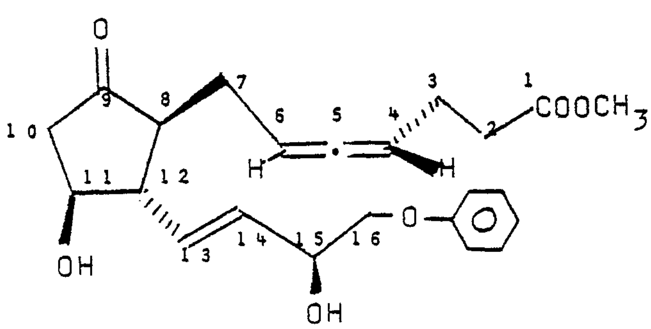

(Ib)

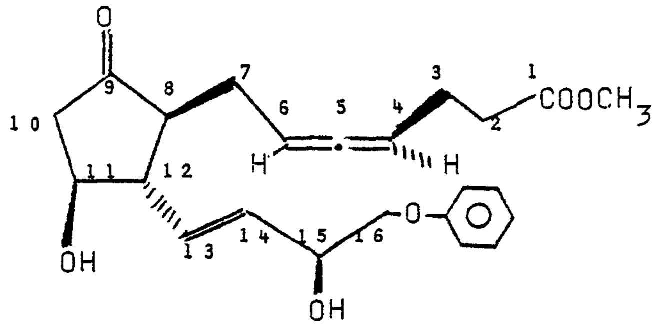

(Ib')

Mixtures that are not racemic, or individual stereoisomers represented by formulas 1a, 1a' 1b and 1b' are covered by this invention.

Enprostil has been shown to be an especially potent gastric secretory inhibitor, and is effective for treatment of ulcers. However, when used in the process of this invention, enprostil will produce the claimed effects even if no ulcers are present.

It has been found that enprostil given in dosages of less than 150 µg reduces cholesterol, GIP and insulin while delaying peak serum glucose levels.

EXAMPLES

The following non-limiting examples will further clarify the nature of this invention.

EXAMPLE I

This Example shows the effect of orally administered enprostil on serum cholesterol.

A group of 20 patients with high cholesterol baseline were orally given 70 µg enprostil twice daily. After five weeks of administration eight showed no change, eleven showed greater than 10 percent reduction, and one showed greater than 25 percent reduction. The median change for the group was 16 percent reduction, with 60 percent of the patients tested showing a significant reduction in serum cholesterol.

A second group of 16 patients with a high cholesterol baseline were given 35 µg enprostil twice daily. After five weeks of administration seven showed no change, eight showed greater than 10 percent serum cholesterol reduction and one showed greater than 25 percent reduction. The median change was eleven percent reduction and 56 percent the patients showed serum cholesterol reduction.

A third group of 13 individuals were given a placebo. After five weeks one showed an increase in cholesterol, and eleven showed no change, and one showed a 10 percent decrease. The median change was a 2 percent increase in cholesterol.

EXAMPLE II

This example shows the effect of orally administered enprostil on serum cholesterol levels.

Table 1 shows the results of a study of sixty five patients given enprostil in doses ranging from 7 µg BID to 70 µg BID to reduce cholesterol compared to 27 subjects who received placebo.

TABLE 1

| | Amount of enprostil administered: | | | |
|---|---|---|---|---|
| | Placebo | 70 µg | 35 µg | 7 µg |
| Cholesterol % decrease (combined 2-12 week of dosing) | 15% | 64% | 64% | 67% |
| Median % change | 0% | -17% | -13% | -11% |
| Number of subjects | 27 | 28 | 28 | 9 |

To further clarify the nature of these changes in a prospective manner, normal subjects were dosed with enprostil for periods ranging from 8 days up to 44 days in duration.

In a 9-day placebo controlled cross-over study, with each subject serving as his own control, the mean reduction in cholesterol, Low Density Lipoprotein (LDL) cholesterol, and High Density Lipoprotein (HDL) cholesterol are shown in Table 2. There was essentially no change in total triglycerides.

TABLE 2

| | CONTROL | ENPROSTIL |
|---|---|---|
| Mean reduction of cholesterol | -1.2% | -15.0% |
| Mean reduction of LDL cholesterol | +1.7% | -21.0% |
| Mean reduction of HDL cholesterol | -7.8% | -9.2% |
| Mean reduction of apoprotein A | -5.3% | -1.8% |
| Mean reduction of apoprotein B | -1.6 | -16.0% |

These findings are surprising since the subjects in this study were normocholesterolemic at baseline (that is serum cholesterol under 250 mg/dl) and were on a free diet with no attempt to regulate dietary cholesterol intake.

Elevated total serum cholesterol levels and LDL-cholesterol levels are known to be atherogenic. It is known that for every 1% reduction in cholesterol there may be as much as a 2% reduction in myocardial morbidity and mortality, JAMA, 251, p 365-374, (1984). It appears that adverse cardiac events are reduced by reduction in total cholesterol as well as LDL cholesterol in an American population.

This example is of further significance in that it showed that enprostil is significantly affecting the apoprotein-B component of the lipoprotein molecule. Apoprotein-B is primarily associated with LDL cholesterol as a protein carrier.

EXAMPLE III

This example shows the effect of orally administered enprostil on serum cholesterol levels in a long term study.

In a 44 day dosing study 20 subject participated in a placebo controlled trial. Ten subjects received enprostil and the remainder received placebo. Data was obtained from 9 of the 10 subjects who received enprostil Six of these nine subjects showed a reduction of total cholesterol by 16 days. Three of the individuals demonstrated a reduction in total and LDL cholesterol throughout the entire 44 day period. In the most dramatic example, subject number 120, total serum cholesterol was reduced from a mean baseline of 208 mg/dl to 123 mg/dl at day 44, which corresponds to a 43% reduction. Similarly there was a 46% reduction in her LDL cholesterol from 140 mg/dl to 75 mg/dl. This subject was normocholesterolemic at baseline and no dietary manipulation was attempted.

EXAMPLE IV

This example shows the effect of orally administered enprostil on serum GIP levels. Serum GIP levels are known to increase after meals.

Groups of 10 subjects participated in a double blind, crossover study. Either the placebo or 70 µg enprostil was orally given 30 minutes before meal time and levels of GIP were measured. 60 minutes after the meal stimulus the recipients of the placebo showed a four fold increase in serum GIP. However, subjects given enprostil showed no increase in GIP 60 minutes after the meal stimulus.

EXAMPLE V

This example shows the effect of orally administered enprostil on serum insulin and glucose levels.

A group of 10 subjects participated in a double blind crossover study. Either a placebo or enprostil was orally given 30 minutes before meal time and levels of insulin and glucose were measured after the meal.

Insulin levels increased five fold in placebo subjects, but only two fold in subjects receiving enprostil. In these same subjects there was no statistically or clinically significant raise in glucose levels in either group of patients. Therefore, enprostil held the levels of glucose at normal while lowering insulin levels.

EXAMPLE VI

This example shows the effect of orally administered enprostil on serum insulin and glucose levels.

Eight healthy male subject received either 70 µg enprostil per day or placebo for eight days followed by a washout period of one week and a subsequent second eight-day administration during which they received the opposite drug regime.

On day 1 and day 8 of each study period the patients reported to the clinical studies unit fasting and were administered 70 µg of enprostil orally in a glass of water. Thirty minutes later they were administered a standard breakfast and blood samples were drawn at -30, just prior to enprostil administration, 0, at the time of enprostil administration, 15, 30, 60, 90, 120 and 180 minutes.

Peak serum glucose was measured between 30 and 60 minutes during the placebo phase. This was delayed to between 60 and 90 minutes while the subjects received enprostil. Furthermore, the area under the serum glucose by

time curve was reduced by approximately 15-20% compared to the placebo phase. These findings were present on both day 1 and day 8 of administration during each study phase.

Accompanying these findings was a marked reduction in serum insulin concentrations for each time point present on both day 1 and 8 as well as essentially complete inhibition of serum GIP for both study days following enprostil administration.

EXAMPLE VII

This example shows the effect of orally administered enprostil on serum carbohydrate levels.

10 normal subjects participated in a 10-day placebo controlled cross-over receiving either placebo or 70 μg enprostil per day. On day 1 and day 9 of each study phase they orally received 5 g of d-xylose, a sugar not normally metabolized in man. Blood measurements for serum xylose were made at -30, 0, +30, 60, 120, 180, 240, and 300 minutes following ingestion of xylose. On day 2 and day 10 of each study phase, the subjects received a 25 g intravenous glucose tolerance test, 30 minutes following enprostil administration.

There was a marked suppression and delay to peak serum xylose levels on both days 1 and 9 of enprostil administration. This was accompanied by a 30% reduction in the amount of xylose excreted over 5 hours: 1.3 g versus 1.8g.

There were no marked differences in the serum glucose by time curves following intravenous glucose administration to subjects between the placebo and the enprostil groups.

Enprostil both delays and retards the absorption of carbohydrates. This is a nonspecific effect since d-xylose is essentially a non-metabolizable sugar and is excreted intact in the human. The ability to suppress rapid absorption of glucose may facilitate improved metabolism of sugar by the liver as well as peripheral utilization with improvement and control of blood sugar profiles in diabetics who are both requiring insulin as well those who require oral pills for a regulation of the diabetes mellitus.

EXAMPLE VIII

This example shows the effect of orally administered enprostil on serum glucose levels.

20 subjects participated in a study lasting up to 44 days. On day -8, all 20 subjects received a placebo capsule followed 30 minutes later by a standard breakfast with serial blood measurements. Eight days later all 20 subjects were randomized to either enprostil 35 μg BID or placebo and continued dosing for a period of 30 days. On days 1, 8, and 30, they were given enprostil followed 30 minutes later by a standard breakfast with serial blood measurements.

Mean serum glucose curves were modified. There was a delay from approximately 60 minutes to 90 minutes in the appearance of peak blood glucose levels which were substantially reduced from the peak blood glucose curves which were measured either in those subjects who received placebo throughout the entire study or in the active control group during the placebo dosing on day -8.

This further confirms that enprostil suppresses and delays the absorption and utilization of carbohydrates, particularly glucose.

**Claims**

1. The use of a compound in the form of a stereoisomer or mixture thereof having the formula:

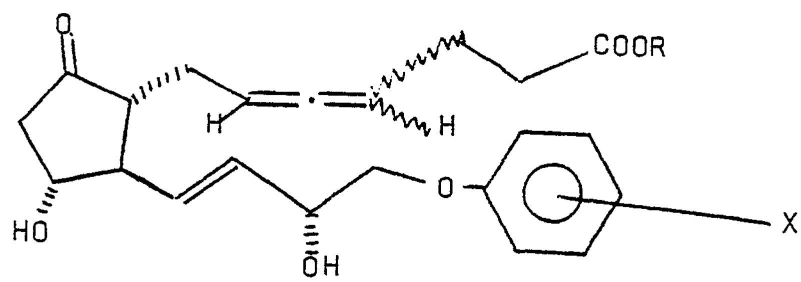

wherein R is hydrogen, lower alkyl; X is hydrogen, halo, trifluoromethyl, lower alkyl or lower alkoxy, and one wavy line is α when the other is β or a pharmaceutically acceptable salt thereof,

for the manufacture of a medicament suitable for reducing serum cholesterol in a disease state.

2. The use of Claim 1 wherein the compound is enprostil.

3. The use of Claim 1 or 2 wherein the effective dose is less than 150 μg a day.

4. The use of any one of Claims 1 to 3 wherein the disease state is hypercholesterolemia or atherosclerosis.

5. The use of a compound in the form of a stereoisomer or mixture thereof having the formula:

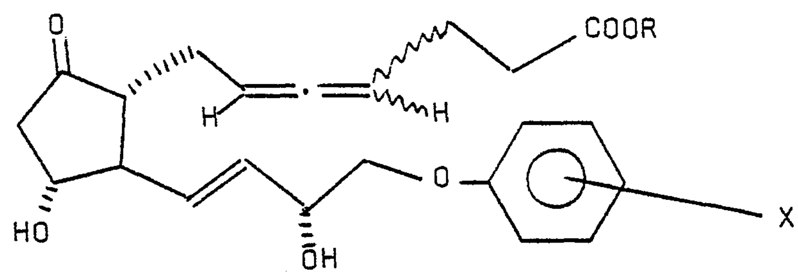

wherein R is hydrogen, lower alkyl; X is hydrogen, halo, trifluoromethyl, lower alkyl or lower alkoxy, and one wavy line is α when the other is β or a pharmaceutically acceptable salt thereof,

for the manufacture of a medicament suitable for delaying and suppressing peak serum glucose levels following carbohydrate load in a disease state.

6. The use of a compound in the form of a stereoisomer or mixture thereof having the formula:

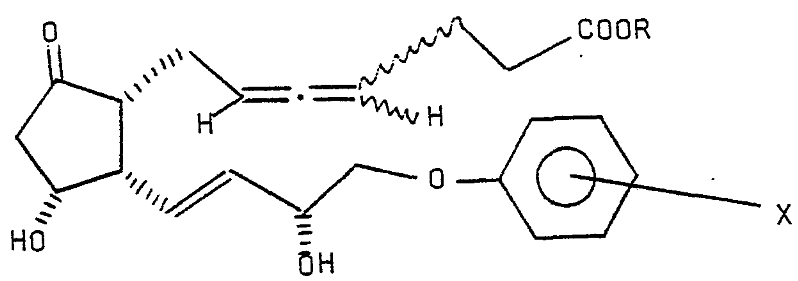

wherein R is hydrogen, lower alkyl; X is hydrogen, halo, trifluoromethyl, lower alkyl or lower alkoxy, and one wavy line is α when the other is β or a pharmaceutically acceptable salt thereof,

for the manufacture of a medicament suitable for reducing serum GIP levels in a disease state.

7. The use of Claim 5 or 6 wherein the compound is enprostil.

8. The use of any one of Claims 5 to 7 wherein the effective dose is between 50 and 150 μg a day.

9. The use of any one of Claims 5 to 8 wherein the disease state is reactive hypoglycemia, non-insulin requiring diabetes, insulin requiring diabetes, nesidioblastosis or pancreatic islet cell hyperplasia.

10. The use of a compound in the form of a stereoisomer or mixture thereof having the formula:

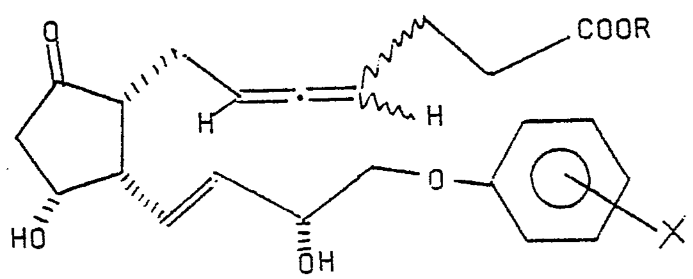

wherein R is hydrogen, lower alkyl; X is hydrogen, halo, trifluoromethyl, lower alkyl or lower alkoxy, and one wavy line is α and the other is β,

for the manufacture of a medicament suitable for preventing and treating atherosclerosis in a disease state.

11. The use of Claim 10 wherein the compound is enprostil.

12. The use of Claim 10 or 11 wherein the effective dose is less than 150 μg a day.